# EUROPÄISCHE PATENTSCHRIFT

(11) **EP 2 485 806 B1**
(45) Veröffentlichungstag und Bekanntmachung des Hinweises auf die Patenterteilung: **31.07.2019**
(21) Anmeldenummer: 10744887.0
(22) Anmeldetag: 13.08.2010
(51) Int. Cl.: A61K 8/35, A61K 8/40, A61K 8/46, A61Q 17/04

(54) **VERWENDUNG VON CETEARYLSULFOSUCCINATEN IN SONNENSCHUTZMITTELN**
USE OF CETEARYL SULFOSUCCINATES IN SUNSCREEN PREPARATIONS
UTILISATION DE CÉTÉARYLSULFOSUCCINATES DANS DES PRÉPARATIONS COSMÉTIQUES DE PROTECTION SOLAIRE

(30) Priorität: 07.10.2009 DE 102009048557
(43) Veröffentlichungstag der Anmeldung: 15.08.2012
(73) Patentinhaber: BASF SE, 67056 Ludwigshafen am Rhein (DE)
(72) Erfinder: SKUBSCH, Kerstin, 25497 Prisdorf (DE); TESCH, Mirko, 22303 Hamburg (DE); KOEHLER, Manuela, 22147 Hamburg (DE); MEYER, Christiane, 20357 Hamburg (DE)
(74) Vertreter: BASF IP Association
(86) Internationale Anmeldenummer: PCT/EP2010/004990
(87) Internationale Veröffentlichungsnummer: WO 2011/042087

(56) Entgegenhaltungen:
- EP-A1- 2 179 986
- WO-A1-2010/046048
- US-A1- 2008 188 574
- MELANIE D PALM ET AL: "Update on photoprotection", DERMATOLOGIC THERAPY, Bd. 20, Nr. 5, 1. September 2007 (2007-09-01), Seiten 360-376, XP055051930, ISSN: 1396-0296, DOI: 10.1111/j.1529-8019.2007.00150.x

## Beschreibung

Die vorliegende Erfindung betrifft die Verwendung von Cetearylsulfosuccinaten in kosmetischen Zubereitungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA- Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen. Ein Hauptnachteil UV-Filter enthaltender kosmetischer Zubereitungen ist, insbesondere wenn es sich um Öl-in-Wasser-Emulsionen (O/W-Emulsionen) handelt, deren Wasserfestigkeit. O/W- Emulsionen sind aufgrund ihrer angenehmen Sensorik (wenig fettig, schnell einziehend) bei den Verbrauchern besonders beliebt. Aufgrund der Tatsache, dass bei O/W-Emulsionen die wässrige Phase die äußere Phase bildet, sind diese Produkte besonders leicht mit Wasser von der Haut abwaschbar. Nach dem Stand der Technik ist es daher erforderlich, Sonnenschutzmitteln auf der Basis einer O/W-Emulsion Filmbildner zuzusetzen, um die Wasserfestigkeit zu erhöhen. Filmbildner führen jedoch dazu, dass die Zubereitung einen klebrig-fettigen Eindruck auf der Haut hinterlassen, schwerer in die Haut einziehen und der Haut einen fettigen Glanz verleihen. Nicht zuletzt sind diese Zubereitungen schwerer verteilbar.

Aus der US 2008/188574 A1 sind Sonnenschutzmittel bekannt, die hydrophob behandelte pulverförmige anorganische Metalloxide, Sulfosuccinate und Wasser enthalten.

Es war daher die Aufgabe der vorliegenden Erfindung, kosmetische Zubereitungen zu entwickeln, die eine höhere Wasserfestigkeit aufweisen, ohne dass die sensorischen Eigenschaften darunter leiden.

Ein weiterer Nachteil herkömmlicher Sonnenschutzmittel auf der Basis von O/W-Emulsionen besteht in dem Umstand, dass man diesen Zubereitungen eine relativ große Menge an teuren und meist schwer löslichen und daher schwierig einzuarbeitenden UV-Filter enthalten muss, damit die Zubereitungen einen ausreichenden UV-Schutz (SPF) bieten. Es war daher die Aufgabe der vorliegenden Erfindung, eine kosmetische Zubereitung zu entwickeln, die mit relativ geringen Konzentrationen an UV-Filtern einen hohen Lichtschutzfaktor (SPF) bietet.

Überraschend werden die Aufgaben gelöst durch die Verwendung von Cetearylsulfosuccinaten zur Erhöhung der Wasserfestigkeit kosmetischer Zubereitungen, welche 2-Ethylhexyl-2-cyano-3,3-diphenylacrylat (Octocrylen) und 4-(tert.-Butyl)-4'-methoxydi- benzoylmethan enthalten, und die Verwendung von Cetearylsulfosuccinaten zur Erhöhung des Lichtschutzfaktors (SPF) kosmetischer Zubereitungen, welche 2-Ethylhexyl-2-cyano-3,3-diphenylacrylat (Octocrylen) und 4-(tert.-Butyl)-4'-methoxydibenzoylmethan enthalten.

Die Zubereitungen mit den erfindungsgemäß verwendeten Cetearylsulfosuccinaten sind einfach und kostengünstig mit den üblichen Herstellungsverfahren herstellbar. Die Zubereitungen weisen ein ausgesprochen angenehmes Hautgefühl auf. Sie sind farb-, lager- und temperaturstabil sowie photostabil.

Die in dieser Beschreibung der Erfindung als mit "erfindungsgemäß", "erfindungsgemäß vorteilhaft" etc. gekennzeichneten Angaben beziehen sich auf die erfindungsgemäße Verwendung.

Zwar kennt der Stand der Technik die Firmenpublikation *"*Eumulgin® Prisma, As diverse as the colors of the light" (2009) der Firma Cognis, doch konnte diese Schrift nicht den Weg zur vorliegenden Erfindung weisen, da sie nicht die erfindungsgemäß verwendete UV-Filterkombination offenbart, keine Aussagen zur Wasserfestigkeit enthält und den Emulgator als besonders geeignet für Zubereitungen beschreibt, die einen hohen Anteil an UV-Filtern enthalten.

Die Erhöhung der Wasserfestigkeit war insbesondere deshalb überraschend, da die Cetearylsulfosuccinate, die erfindungsgemäß bevorzugt in Form von Dinatriumsalzen eingesetzt werden, selbst eine gut wasserlösliche Substanz darstellen. Eine verbesserte Wasserfestigkeit auch gegenüber Emulgatorsystemen, die nichtionische Emulgatorbestandteile (beispielsweise Glycerylstearat) enthalten, war daher für den Fachmann nicht zu erwarten.

Das Cetearylsulfosuccinat wird erfindungsgemäß vorteilhaft als Dinatriumsalz eingesetzt. Es handelt sich bei diesem Rohstoff vorteilhaft um eine Mischung aus Dinatriumcetylsulfosuccinat: 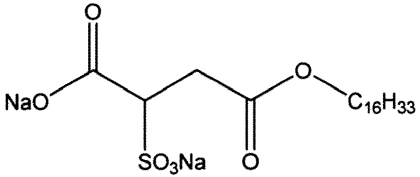 und
Dinatriumstearylsulfosuccinat:

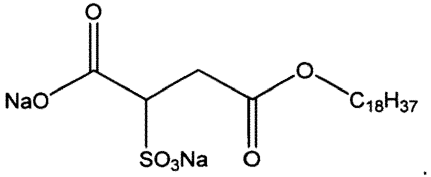

Diese Komponente ist beispielsweise bei der Firma Cognis unter dem Handelsnamen *Eumulgin*® *Prisma* erhältlich.

Es ist erfindungsgemäß vorteilhaft, wenn die Verwendung dadurch gekennzeichnet ist, dass die Zubereitung Cetearylsulfosuccinate in einer Konzentration von 0,05 bis 2 Gewichts-%, bezogen auf das Gesamtgewicht der Zubereitung, enthält.

Es ist erfindungsgemäß bevorzugt, wenn die Verwendung dadurch gekennzeichnet ist, dass die Zubereitung Cetearylsulfosuccinate in einer Konzentration von 0,1 bis 1 Gewichts-%, bezogen auf das Gesamtgewicht der Zubereitung, enthält.

Es ist erfindungsgemäß vorteilhaft, wenn in der Zubereitung 2-Ethylhexyl-2-cyano-3,3-diphenylacrylat (Octocrylen) in einer Konzentration von 0,5 bis 20 Gewichts-%, bezogen auf das Gesamtgewicht der Zubereitung, verwendet werden.

Es ist erfindungsgemäß bevorzugt, wenn in der Zubereitung 2-Ethylhexyl-2-cyano-3,3-diphenylacrylat (Octocrylen) in einer Konzentration von 1 bis 10 Gewichts-%, bezogen auf das Gesamtgewicht der Zubereitung, verwendet werden.

Es ist erfindungsgemäß vorteilhaft, wenn in der Zubereitung 4-(tert.-Butyl)-4'-methoxydibenzoylmethan in einer Konzentration von 0,05 bis 10 Gewichts-%, bezogen auf das Gesamtgewicht der Zubereitung, verwendet werden.

Es ist erfindungsgemäß bevorzugt, wenn in der Zubereitung 4-(tert.-Butyl)-4'-methoxydibenzoylmethan in einer Konzentration von 1 bis 5 Gewichts-%, bezogen auf das Gesamtgewicht der Zubereitung, verwendet werden.

Es ist erfindungsgemäß vorteilhaft, wenn die Zubereitung einen oder mehrere weitere UV-Filter gewählt aus der Gruppe der Verbindungen Phenylen-1,4-bis-(2-benz-imidazyl)-3,3'-5,5'-tetrasulfonsäuresalze; 2-Phenylbenzimidazol-5-sulfonsäuresalze; 1,4-di(2-oxo-10-Sulfo-3-bornyliden-methyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-{6-{2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4 Methylbenzyliden)campher; 3-Benzylidencampher; Ethylhexylsalicylat; 2-(4'-Diethylamino-2'-hydoxybenzoyl)-benzoesäure-hexylester; Terephthalidendicamphersulfonsäure; 4-(Dimethylamino)-benzoesäure(2-ethylhexyl) ester; 4-(Dimethylamino)benzoesäureamylester; 4-Methoxybenzalmalonsäuredi(2-ethylhexyl) ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'- methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan/Dimethylsiloxan - Copolymer; Dioctylbutylamidotriazon (1NCI: Diethylhexyl-Butamidotriazone); 2,4-bis-[5-1(dimethylpropyl)benzoxazol-2-yl-(4-phenyl)-imino]-6-{2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (1NCI: Ethylhexyl Triazone); 2,4-Bis-{(4-(2-ethylhexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate enthält.

Die erfindungsgemäß verwendeten vorteilhaften Merocyanine werden dabei gewählt aus der Gruppe der Verbindungen

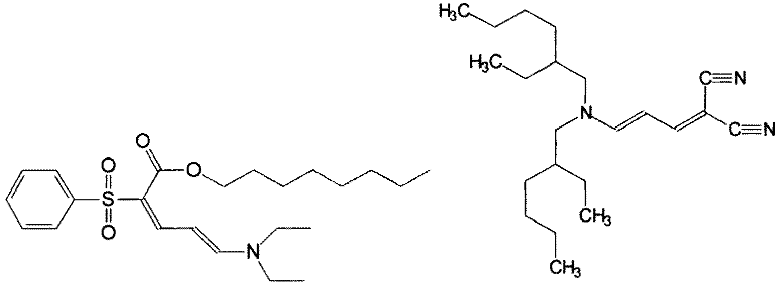

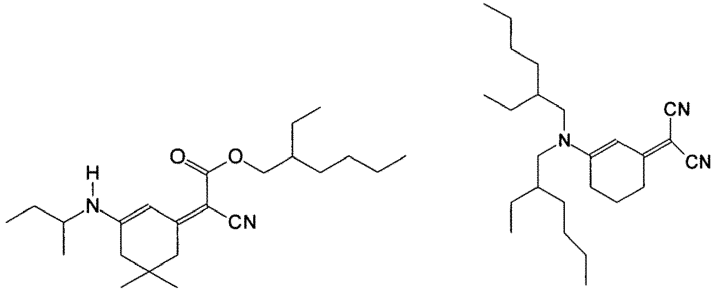

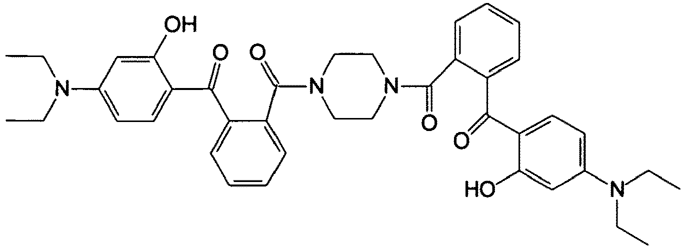

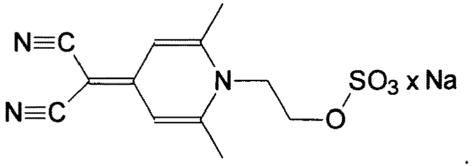

Als erfindungsgemäß vorteilhaftes Piperazinderivat kann dabei die folgende Verbindung eingesetzt werden:

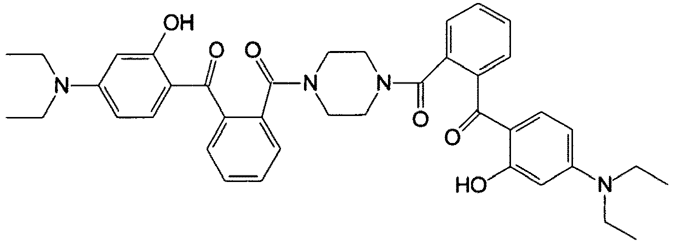

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid AL(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Es ist erfindungsgemäß vorteilhaft, wenn die Zubereitung einen oder mehrere Parfümstoffe enthält.

Die erfindungsgemäß verwendeten Parfümstoffe können beispielsweise gewählt werden aus der Gruppe der Verbindungen 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, d-Limonene, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Hydroxyisohexyl 3-Cyclohexencarboxaldehyde, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin, Limonen [5989-27-5], Citral, Linalool [78-70-6), alpha-Isomethylionon [1335-46-2), Geraniol [106-24-1), Citronellol [106-22-9), [24851-98-7), [18479-58-8), [54464-57-2], [80-54-6), [1222-05,5), [32388-55-9), [105-95-3), [31906-04-4), [8008-57-9], [32210-23-4], [120-57-0], [115-95-7), [101-86-0), [140-11-4], [6259-76-3] und [127-51-5].

Besonders vorteilhafte Ausführungsformen der vorliegenden Erfindung sind dadurch gekennzeichnet, dass die Zubereitung einen oder mehrere Wirkstoffe gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1, 16-dicarbonsäure, Polydocanol, enthält.

Vorteilhafte Ausführungsformen der vorliegenden Erfindung sind dadurch gekennzeichnet, dass die Zubereitung ein oder mehrere Verbindungen gewählt aus der Gruppe der Parabene Phenoxyethanol, Ethylhexylglycerin, 2-Methylpropan-1,3-diol, Butylenglycol, Propylenglycol, Pentan-1,2-diol, Hexan-1,2-diol, Caprylylglycol enthält.

Neben den erfindungsgemäßen bzw. erfindungsgemäß vorteilhaften Bestandteilen, kann die Zubereitung die üblichen Bestandteile wässriger und lipophiler Phasen einer kosmetischen O/W-Emulsion enthalten.

Die Wasserphase der Zubereitungen kann beispielsweise vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether wie Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Verdickungsmittel, welches oder welche beispielsweise vorteilhaft gewählt werden können aus der Gruppe Siliciumdioxid, Aluminiumsilikate, Polysaccharide bzw. deren Derivate, z. B. Hyaluronsäure, Xanthangummi, Carragenanen, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCi-Bezeichnung Acrylates/C10-30 Alkyl Acrylate Crosspolymer (z. B. Pemulen TR 1, Pemulen TR 2, Carbopol 1328 von der Fa. NOVEON), Ultrathix P 100 von der Firma ISP (INCi: Acrylic AcidNP Crosspolymer) sowie Aristoflex AVC (INCi: Ammonium AcryloyldimethyltaurateNP Copolymer) und ARISTOFLEX HMB (Ammonium Acryloyldimethyltaurate/Beheneth-25 Methacrylate Copolymer).

Die Ölphase der Zubereitung wird beispielsweise vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. 8. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-0ctyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z.B. Jojobaöl.

Ferner kann die Ölphase vorteilhaft gewählt werden aus der Gruppe der Dialkylether und Dialkylcarbonate, vorteilhaft sind z.B. Dicaprylylether (*Cetiol* OE) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der Formulierungen einen Gehalt an C₁₂₋₁₅-Alkylbenzoat aufweist oder vollständig aus diesem besteht.

Vorteilhafte Ölkomponenten sind ferner z.B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar* AB) und/oder Diethylhexylnaphthalat *(Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan, Polyolefine, hydrogenierte Polyisobutene, C13-16 Isoparaffin und Isohexadecan. Unter den Polyolefinen sind Polydecene die bevorzugten Substanzen.

Die Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Vorteilhafte in Form sphärischer Puder vorliegende Siloxanelastomere sind die mit der INCi-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Die Zubereitung kann ein oder mehrere der üblichen Filmbildner zur Erhöhung der Wasserfestigkeit enthalten. Es lassen sich jedoch auch vorteilhafte Zubereitungen ohne diese Filmbildner herstellen.

Vorteilhafterweise weist die Zubereitung einen pH-Wert von 5 bis 8 auf. Dieser kann durch die herkömmlichen Säuren, Basen und Puffersysteme eingestellt werden.

Zur Anwendung werden die kosmetischen Zubereitungen in der für Kosmetika üblichen Weise auf die Haut und/oder die Haare in ausreichender Menge aufgebracht Die Zubereitung kann sprühbar sein.

Vorteilhafterweise wird die Zubereitung in Kunststoffflaschen aus PE, PP, PET oder Verbundstoffen aufbewahrt.

Der nachfolgende Vergleichsversuch zeigt exemplarisch die erfindungsgemäßen Effekte:

### Vergleichsversuch

Es wurden die folgenden Rezepturen hergestellt und
a) die Wasserfestigkeit mit Hilfe der Kontaktwinkelmessung (siehe "Contact angle measurement-a reliable supportive method for screening water-restistance of ultravioletprotecting products in vivo" Intern. Journal of Cosmetic Science, 2007, 29, 283-291) sowie
b) der in vivo Lichtschutzfaktor (SPF) bestimmt.

| | **Zusammensetzung** | **A** | **B** |
|---|---|---|---|
| Emulgatormischung | Tegin VS + Emulgade F | | 2,65 |
| Emulgator | Disodium Cetearyl Sulfosucinnate | 0,25 | |
| UVA-Filter | Butyl Methoxydibenzoylmethane | 4,5 | 4,5 |
| UVB-Filter | Octocrylene | 4,5 | 4,5 |
| Lichtfiltermischung | Ethylhexyl Methoxycinnamate | 11 | 11 |
| | Phenylbenzimidazole Sulfonic Acid | | |
| | Bis-Ethylhexyloxyphenol Methoxyphenyl Triazine | | |
| | Diethylhexyl Butamido Triazone | | |
| | Titanium Dioxide | | |
| | Aqua + Trisodium EDTA | | |
| Konservierung | Methylparaben | 9,4 | 9,4 |
| | Phenoxyethanol | | |
| | Alcohol Denat. | | |
| | Glycerin | | |
| Lipophile Phase | Octyldodecanol | 18,7 | 18,7 |
| | Myristyl Myristate | | |
| | Tocopheryl Acetate | | |
| | C12-15 Alkyl Benzoate | | |
| | Butylene Glycol Dicaprvlate/Dicaprate | | |
| | Parfum | | |
| | Cetyl Alcohol | | |
| Verdicker | Xanthan Gum | 0,45 | 0,45 |
| | Acrylates/C10-30 Alkyl Acrylate Crosspolymer | | |
| | Tapioca Starch | 1 | 1 |
| Wasserphase | 45%- ige Natronlauge | q.s. | q.s. |
| | Glycerin | 0,9 | 0,9 |
| | VE-Wasser | ad 100 | ad 100 |
| | **in-vivo SPF** | **96** | **67** |
| | **Kontaktwinkel** (je höher desto wasserfester) | **56°** | **23°** |

**Fazit:** Die Zusammensetzung ist deutlich wasserfester und hat einen höheren Lichtschutzfaktor.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Emulsion** | **1** | **2** | **3** | **4** |
|---|---|---|---|---|
| Disodium Cetearyl Sulfosucinnate | 0,5 | 0,25 | 0,5 | 1 |
| Cetearylalkohol | | | 1 | |
| Stearylalkohol | 0,5 | | | |
| Cetylalkohol | | 2,2 | | |
| Myristylmyristat | 1 | 1,5 | | |
| Acrylates/C₁₀₋₃₀ Alkyl Acrylat Crosspolymer | 0,1 | 0,05 | | |
| Copolymer aus Vinylpyrrolidon und Acrylsäure | | | 0,5 | |
| Xanthan Gum | 0,4 | 0,3 | | |
| Natrium Polyacrylat | | | | 0,2 |
| Hydriertes Coca-Glycerid | 1 | | | |
| C₁₂₋₁₅ Alkyl Benzoat | 5 | 8 | 4 | 5 |
| Dicaprylyl Caprate | 2 | | | |
| Butylenglykol DicaprylaUDicaprat | 5 | 4 | 7 | |
| Propylheptyl Caprylate | 5 | 5 | | 12 |
| Dicaprylyl Ether | | | 5 | 2 |
| Octyldodecanol | | 2 | | |
| Tapioka Stärke | | 1 | | |
| Cyclomethicon | | 3 | 5 | |
| Dimethicon | | | 1 | |
| VP/Hexadecene Copolymer | 0,5 | | 0,2 | |
| Propylenglykol | | 1 | | 5 |
| Glycerin | 9 | 3 | 2 | 3 |
| Octan-1,2-diol | | 5 | 2 | |
| Octocrylen | 9 | 2,5 | 1 | 9 |
| Butyl Methoxydibenzoylmethan | 4,5 | 2 | 1 | 5 |
| Bis-Ethylhexyloxyphenol Methoxyphenyl Triazine | 3,5 | 2 | | |
| Phenylbenzimidazol Sulfonsäure | | 1 | | |
| Tris-biphenyltriazain | | | 2 | |
| Ethylhexyltriazin | | | | 3 |
| Ethylhexylmethoxycinnamat | 1 | | | |
| Dioctylbutylamidotriazin | 1 | | | |
| Titandioxid + Trimethoxycaprylylsilane | 3,5 | 2 | | |
| Vitamin E Acetat | 0,1 | 0,2 | 0,3 | 0,1 |
| Na₂H₂EDTA | 0,1 | 0,2 | 0,2 | 0,2 |
| Parfüm | 0,4 | 0,3 | 0,4 | 0,25 |
| Alkohol | 4 | 6 | | |
| Lauroylarginat | | 0,1 | 0,4 | 0,5 |
| Phenoxyethanol | 0,7 | 1 | 0,5 | |
| Benzethoniumchlorid | 0,05 | | | |
| Piroctone Olamine | | | 0,05 | |
| Ethylhexylglycerin | | | | 1 |
| Natriumhydroxid | q.s. | q.s. | q.s. | q.s. |
| Wasser | ad 100,0 | ad 100,0 | ad 100,0 | ad 100,0 |

| **Emulsion** | **5** | **6** | **7** | **8** |
|---|---|---|---|---|
| Disodium Cetearyl Sulfosucinnate | 0,3 | 0,5 | 0,2 | 0,8 |
| Sucrosepolystearat | | | | 1 |
| Stearylalkohol | 0,5 | | | |
| Cetylalkohol | | 2,2 | | |
| Myristylmyristat | 1 | 1,5 | | |
| Acrylates/C₁₀₋₃₀ Alkyl Acrylat Crosspolymer | 0,1 | 0,05 | | 0,3 |
| Copolymer aus Vinylpyrrolidon und Acrylsäure | | | 1,0 | |
| Xanthan Gum | 0,4 | 0,3 | | |
| Hydriertes Coca-Glycerid | 1 | | | |
| C₁₂₋₁₅ Alkyl Benzoat | | 8 | 4 | 5 |
| Dicaprylyl Caprate | 2 | | | |
| Butylenglykol DicaprylaUDicaprat | | 4 | 7 | |
| Propylheptyl Caprylate | 5 | 5 | | |
| Dicaprylyl Ether | | | 5 | 2 |
| Octyldodecanol | | 2 | | |
| Mais Stärke | 2 | | | |
| Cyclomethicon | | 3 | 5 | |
| Dimethicon | | | 1 | |
| VP/Hexadecene Copolymer | 0,5 | | 0,2 | |
| Glycerin | 9 | 3. | 2 | 3 |
| Octocrylen | 2 | 4 | 3 | 10 |
| Butyl Methoxydibenzoylmethan | 5 | 4 | 5 | 5 |
| Diethylamino Hydroxybenzoyl Hexyl Benzoat | 2 | | | |
| Phenylbenzimidazol Sulfonsäure | 3 | | | |
| Zinkoxid | | 3 | | |
| Octylsalicylat | 5 | | | |
| Dimethicon / Vinyl Dimethicon Crosspolymer | 5 | | 3 | |
| Vitamin E Acetat | 0,1 | 0,2 | 0,3 | 0,1 |
| Na₂H₂EDTA | 0,1 | 0,2 | 0,2 | 0,2 |
| Alkohol | 4 | 6 | | |
| Parfüm | q.s. | q.s. | q.s. | q.s. |
| Konservierungsmittel | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

## Patentansprüche

1. Verwendung von Cetearylsulfosuccinaten zur Erhöhung der Wasserfestigkeit kosmetischer Zubereitungen, welche 2-Ethylhexyl-2-cyano-3,3-diphenylacrylat (Octocrylen) und 4- (tert.-Butyl)-4'-methoxydibenzoylmethan enthalten.

2. Verwendung von Cetearylsulfosuccinaten zur Erhöhung des Lichtschutzfaktors (SPF) kosmetischer Zubereitungen, welche 2-Ethylhexyl-2-cyano-3,3-diphenylacrylat (Octocrylen) und 4-(tert.-Butyl)-4'-methoxydibenzoylmethan enthalten.

3. Verwendung nach Anspruch 1 oder 2, **dadurch gekennzeichnet, dass** die Zubereitung einen oder mehrere weitere UV-Filter gewählt aus der Gruppe der Verbindungen Phenylen-1,4-bis-(2-benzimidazyl)-3,3'-5,5' tetrasulfonsäuresalze; 2 Phenylbenzimidazol-5-sulfonsäuresalze; 1,4 di(2 oxo-10-Sulfo-3-bomylidenmethyl)-Benzol und dessen Salze; 4-(2-Oxo-3-bornylidenmethyl)benzol-sulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy] disiloxanyl]propyl]-phenol; 3-(4-Methylbenzyliden)campher; 3-Benzyliden-campher; Ethylhexylsalicylat; 2-(4'-Diethylamino-2'-hydoxybenzoyl)-benzoesaurehexylester; Terephthalidendicamphersulfonsäure; 4-(Dimethylamino)-benzoesäure(2-ethylhexyl)ester; 4-(Dimethylamino)benzoesäure-amyl-ester; 4-Methoxybenzalmalonsäuredi(2-ethylhexyl)ester; 4-Methoxyzimtsäure(2-ethyl-hexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy- 4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)-propenyl)-methoxysiloxan/Dimethylsiloxan-Copolymer; Dioctylbutylamidotriazon (INCI: Diethylhexyl-Butamidotriazone); 2,4-bis-[5-1 (dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexytester) (auch: 2,4,6-Tris-[anilino-(p-carbo-2'-ethyl-1'-hexytoxy)]-1,3,5-triazin (INCI: Ethylhexyl Triazone); 2.4-Bis-{[4-(2-ethyl-hexyloxy)-2-hydroxyJ-phenyl}-6-{4-metho:xyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylamino-benzoat)-s-triazin, 4-Dicyanomethylen-2,6-dimethyl-1,4-dihydropyridin-N-(ethyloxysulfatester-salz), Titandioxid, Zinkoxid, Merocyanine, Piperazinderivate, enthält.

4. Verwendung nach einem der vorhergehenden Ansprüche, **dadurch gekennzeichnet, dass** die Zubereitung einen oder mehrere Parfümstoffe enthält.

5. Verwendung nach einem der vorhergehenden Ansprüche, **dadurch gekennzeichnet, dass** die Zubereitung einen oder mehrere Wirkstoffe enthält, gewählt aus der Gruppe der Verbindungen Tocopherylacetat, Tocopherol, Gylcyrrhetinsäure bzw. dessen Salze, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, β-Alanin, Licochalcon A, Hyaluronsäure, Saponine, Dihydroxyaceton; 8-Hexadecen-1,16-dicarbonsäure, Polydocanol.

6. Verwendung nach einem der vorhergehenden Ansprüche, **dadurch gekennzeichnet, dass** die Zubereitung Cetearylsulfosuccinate in Form des Dinatriumsalzes enthält.

7. Verwendung nach einem der vorhergehenden Ansprüche, **dadurch gekennzeichnet, dass** die Zubereitung Cetearylsulfosuccinate in einer Konzentration von 0,05 bis 5 Gewichts-%, bezogen auf das Gesamtgewicht der Zubereitung, enthält.

8. Verwendung nach einem der vorhergehenden Ansprüche, **dadurch gekennzeichnet, dass** die Zubereitung 2-Ethylhexyl-2-cyano-3,3-diphenytacrylat (Octocrylen) in einer Konzentration von 0,1 bis 2 Gewichts-%, bezogen auf das Gesamtgewicht der Zubereitung, enthält.

9. Verwendung nach einem der vorhergehenden Ansprüche, **dadurch gekennzeichnet, dass** die Zubereitung 4-(tert.-Butyl)-4'-methoxydibenzoylmethan in einer Konzentration von 0,5 bis 5 Gewichts-%, bezogen auf das Gesamtgewicht der Zubereitung, enthält.

## Claims

1. The use of cetearyl sulfosuccinates to increase the water resistance of cosmetic preparations comprising 2-ethylhexyl 2-cyano-3,3-diphenyl acrylate (octocrylene) and 4-(tert-butyl)-4'-methoxydibenzoylmethane.

2. The use of cetearyl sulfosuccinates to increase the sun protection factor (SPF) of cosmetic preparations comprising 2-ethylhexyl 2-cyano-3,3-diphenyl acrylate (octocrylene) and 4-(tert-butyl)-4'-methoxydibenzoylmethane.

3. The use according to claim 1 or 2, wherein the preparation comprises one or more further UV filters selected from the group of the compounds phenylene-1,4-bis(2-benzimidazyl)-3,3'-5,5'-tetrasulfonic acid salts; 2-phenyl benzimidazole-5-sulfonic acid salts; 1,4-di(2-oxo-10-sulfo-3-bornylidenemethyl)benzene and salts thereof; 4-(2-oxo-3-bornylidene methyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; hexyl 2-(4'-diethylamino-2'-hydroxybenzoyl)benzoate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxy-silox ane/dimethylsiloxane copolymer; dioctylbutylamidotriazone (INCI: Diethylhexyl Butamido Triazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris (2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tris(biphenyl)-1,3,5-triazine; 2,4-bis(4'-dineopentylaminobenzalmalonate)-6-(4"-butylaminobenzoate)-s-triazine; 4-dicyanomethylene-2,6-dimethyl-1,4-dihydropyridine-N-(ethyloxysulfate ester salt), titanium dioxide, zinc oxide, merocyanine, piperazine derivatives.

4. The use according to any of the preceding claims, wherein the preparation comprises one or more perfumes.

5. The use according to any of the preceding claims, wherein the preparation comprises one or more active ingredients selected from the group of the compounds tocopheryl acetate, tocopherol, glycyrrhetic acid or salts thereof, alpha-lipoic acid, folic acid, phytoene, D-biotin, coenzyme Q10, alpha-glucosylrutin, carnitine, carnosine, natural and/or synthetic isoflavonoids, glycerylglucose, creatine, creatinine, taurine, β-alanine, licochalcone A, hyaluronic acid, saponins, dihydroxyacetone; 8-hexadecene-1,16-dicarboxylic acid, polidocanol.

6. The use according to any of the preceding claims, wherein the preparation comprises cetearyl sulfosuccinates in the form of the disodium salt.

7. The use according to any of the preceding claims, wherein the preparation comprises cetearyl sulfosuccinates at a concentration of 0.05 to 5% by weight, based on the total weight of the preparation.

8. The use according to any of the preceding claims, wherein the preparation comprises 2-ethylhexyl 2-cyano-3,3-diphenylacrylate (octocrylene) at a concentration of 0.1 to 2% by weight, based on the total weight of the preparation.

9. The use according to any of the preceding claims, wherein the preparation comprises 4-(tert-butyl)-4'-methoxydibenzoylmethane at a concentration of 0.5 to 5% by weight, based on the total weight of the preparation.

## Revendications

1. Utilisation de sulfosuccinates de cétéaryle pour augmenter la résistance à l'eau de préparations cosmétiques qui contiennent de l'acrylate de 2-éthylhexyl-2-cyano-3,3-diphényle (octocrylène) et du 4-(tert.-butyl)-4'-méthoxydibenzoylméthane.

2. Utilisation de sulfosuccinates de cétéaryle pour augmenter le facteur de protection solaire (FPS) de préparations cosmétiques qui contiennent de l'acrylate de 2-éthylhexyl-2-cyano-3,3-diphényle (octocrylène) et du 4-(tert.-butyl)-4'-méthoxydibenzoylméthane.

3. Utilisation selon la revendication 1 ou 2, **caractérisée en ce que** la préparation contient un ou plusieurs filtres UV supplémentaires choisis dans le groupe de composés constitué par les sels de l'acide phénylène-1,4-bis-(2-benzimidazyl)-3,3'-5,5'-tétrasulfonique ; les sels de l'acide 2-phénylbenzimidazole-5-sulfonique ; le 1,4 di(2-oxo-10-sulfo-3-bornylidène-méthyl)-benzène et ses sels ; les sels de l'acide 4-(2-oxo-bornylidène-méthyl)benzène-sulfonique ; les sels de l'acide 2-méthyl-5-(2-oxo-3-bornylidène-méthyl)sulfonique ; le 2,2'-méthylène-bis-(6-(2H-benzotriazol-2-yl)-4-(1,1,3,3-tétraméthylbutyl)-phénol) ; le 2-(2H-benzotriazol-2-yl)-4-méthyl-6-[2-méthyl-3-[1,3,3,3-tétramethyl-1-[(triméthylsilyl)oxy]disiloxanyl]propyl]-phénol ; le 3-(4-méthylbenzylidène)camphre ; le 3-benzylidène-camphre ; le salicylate d'éthylhexyle ; l'ester hexylique de l'acide 2-(4'-diéthylamino-2'-hydoxybenzoyl)-benzoïque ; l'acide téréphtalidène-dicamphre-sulfonique ; l'ester 2-éthylhexylique de l'acide 4-(diméthylamino)-benzoïque ; l'ester amylique de l'acide 4-(diméthylamino)benzoïque ; l'ester di(2-éthylhexylique) de l'acide 4-méthoxybenzalmalonique ; l'ester 2-éthylhexylique de l'acide 4-méthoxycinnamique ; l'ester isoamylique de l'acide 4-méthoxycinnamique ; la 2-hydroxy-4-méthoxybenzophénone ; la 2-hydroxy-4-méthoxy-4'-méthylbenzophénone ; la 2,2'-dihydroxy-4-méthoxybenzophénone ; le salicylate d'homomenthyle ; le 2-hydroxy-benzoate de 2-éthylhexyle ; le benzalmalonate de diméthicodiéthyle ; le copolymère de 3-(4-(2,2-bis-éthoxycarbonylvinyl)-phénoxy)-propényl)-méthoxysiloxane/diméthylsiloxane ; la dioctylbutylamidotriazone (INCI : Diethylhexyl-Butamidotriazone) ; la 2,4-bis-[5-1-(diméthyl-propyl)benzoxazol-2-yl-(4-phényl)-imino]-6-(2-éthylhexyl)-imino-1,3,5-triazine de n° CAS 288254-16-0) ; l'ester tris-(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; la 2,4-bis-{[4-(2-éthyl-hexyloxy)-2-hydroxy]-phényl}-6-{4-méthoxyphényl)-1,3,5-triazine (INCI : Bis-Ethylhexyloxyphenol Methoxyphenyl Triazin) ; la 2,4,6-tris-(biphényl)-1,3,5-triazine ; la 2,4-bis-(4'-dinéopentylaminobenzalmalonate)-6-(4"-butylamino-benzoate)-s-triazine ; le sel de N-(éthyloxysulfate-ester) de 4-dicyanométhylène-2,6-diméthyl-1,4-dihydropyridine ; le dioxyde de titane, l'oxyde de zinc, la mérocyanine, les dérivés de pipérazine.

4. Utilisation selon l'une quelconque des revendications précédentes, **caractérisée en ce que** la préparation contient une ou plusieurs substances parfumées.

5. Utilisation selon l'une quelconque des revendications précédentes, **caractérisée en ce que** la préparation contient un ou plusieurs agents actifs, choisis dans le groupe de composés constitué par l'acétate de tocophéryle, le tocophérol, l'acide glycyrrhizique ou ses sels, l'acide alpha-liponique, l'acide folique, le phytoène, la D-biotine, la coenzyme Q10, l'alpha-glucosylrutine, la carnitine, la carnosine, les isoflavonoïdes naturels et/ou synthétiques, le glycérylglucose, la créatine, la créatinine, la taurine, la β-alanine, la licochalcone A, l'acide hyaluronique, la saponine, la dihydroxyacétone, l'acide 8-hexadécène-1,16-dicarboxylique, le polydocanol.

6. Utilisation selon l'une quelconque des revendications précédentes, **caractérisée en ce que** la préparation contient des sulfosuccinates de cétéaryle sous la forme du sel de disodium.

7. Utilisation selon l'une quelconque des revendications précédentes, **caractérisée en ce que** la préparation contient des sulfosuccinates de cétéaryle en une concentration de 0,05 à 5 % en poids, par rapport au poids total de la préparation.

8. Utilisation selon l'une quelconque des revendications précédentes, **caractérisée en ce que** la préparation contient de l'acrylate de 2-éthylhexyl-2-cyano-3,3-diphényle (octocrylène) en une concentration de 0,1 à 2 % en poids, par rapport au poids total de la préparation.

9. Utilisation selon l'une quelconque des revendications précédentes, **caractérisée en ce que** la préparation contient du 4-(tert.-butyl)-4'-méthoxydibenzoylméthane en une concentration de 0,5 à 5 % en poids, par rapport au poids total de la préparation.
